# EUROPEAN PATENT SPECIFICATION

(11) **EP 0 699 061 B1**
(45) Date of publication and mention of the grant of the patent: **24.03.1999**
(21) Application number: 93918595.5
(22) Date of filing: 03.08.1993
(51) Int. Cl.: A61F 13/15

(54) **ABSORBENT ARTICLE WITH ELASTIC FEATURE HAVING A PRESTRAINED WEB PORTION**
ABSORBIERENDER ARTIKEL MIT EINEM ELASTISCHEN, EIN VORGEDEHNTES GEWEBETEILAUFWEISENDES ELEMENT
ARTICLE ABSORBANT A ELEMENT ELASTIQUE COMPRENANT UNE PARTIE VOILE PRECONTRAINTE

(30) Priority: 28.08.1992 US 937619
(43) Date of publication of application: 06.03.1996
(73) Proprietor: THE PROCTER & GAMBLE COMPANY, Cincinnati, Ohio 45202 (US)
(72) Inventor: BUELL, Kenneth, Barclay, Cincinnati, OH 45215 (US); RICHARDSON, James, William, Cincinnati, OH 45242 (US); BENSON, Douglas, Herrin, West Harrison, IN 47060 (US)
(74) Representative: Bottema, Johan Jan
(86) International application number: US9307274
(87) International publication number: WO9405241

(56) References cited:
- EP-A- 0 396 800
- EP-A- 0 443 082
- WO-A-91/15355
- WO-A-92/22272
- WO-A-93/09742
- US-A- 4 880 682

## Description

### FIELD Of THE INVENTION

The present invention relates to absorbent articles with elastic features, and more particularly, to absorbent articles with elastic features having prestrained nonelastic web portions coinciding with at least a portion of the elastomeric member of the elastic feature. Further, the present invention relates to methods and apparatus for mechanically prestraining a nonelastic web forming a portion of the elastic feature so as to enhance the performance of the elastic feature.

### BACKGROUND of THE INVENTION

Infants and other incontinent individuals wear absorbent articles such as diapers to receive and contain urine and other body exudates. Absorbent articles function both to contain the discharged materials and to isolate these materials from the body of the wearer and from the wearer's garments and bed clothing. However, absorbent articles have a tendency to sag or gap away from and to slide/slip down on the body of the wearer during wear. This sagging/gapping and sliding/slipping is caused by the relative motions of the wearer as the wearer breathes, moves and changes positions, by the downward forces generated when the absorbent article is loaded with body exudates, and by the deformation of the materials of the absorbent article itself when subjected to such wearer's motions. This sagging/gapping and sliding/slipping of the absorbent article can lead to premature leakage and poor fit of the absorbent article about the wearer in the waist regions and the leg regions of the absorbent article.

In order to more snugly fit absorbent articles about the wearer, certain commercially available absorbent articles have been provided with elastic features. An example of a disposable diaper with an elastomeric waist feature which has achieved wide acceptance and commercial success is disclosed in U.S. Patent 4,515,595 issued to Kievit and Osterhage on May 7, 1985. Elastic waist features will typically comprise an elasticized waistband consisting of an elastomeric member contractibly affixed between the topsheet and the backsheet. The elasticized waistband is, thus, designed to expand and contract with the wearer's motions and to maintain the fit of the absorbent article about the waist of the wearer during use. Disposable absorbent articles having elastic leg features are also known to the art. For example, U.S. Patent 3,860,003, entitled "Contractable Side Portions For Disposable Diaper", issued to Buell on January 14, 1975, describes an absorbent article having an elasticized leg cuff which has achieved wide acceptance and commercial success. EP 0 396 800 describes disposable diapers that possess improved elastic characteristics. The elastic elements are secured in a relaxed condition to a web of outer sheet fabric, and then the elastic web is stretched.

However, it has been found that the extension and contraction characteristics of elastic features on absorbent articles are limited or inhibited by the extension/contraction characteristics of the nonelastic webs making up the elastic feature. This problem is particularly encountered when the elastomeric member of the elastic feature comprises a heat shrinkable elastomeric material. The contraction of the heat shrinkable elastomer can be severely limited by the ability (lack thereof) of the nonelastic web(s) making up the elastic feature to contract.

Thus, it would be advantageous to provide an elastic feature for an absorbent article, particularly an elastic waist feature, that provides better extension/contraction characteristics in order to provide improved fit reduced leakage, and wearer comfort.

Therefore, it is an object of the present invention to provide an absorbent article having an elastic feature, particularly an elastic waist feature, wherein one or more of the nonelastic webs forming the elastic feature is mechanically prestrained to enhance the performance of the elastic feature.

It is a further object of the present invention to provide a method and apparatus for mechanically stretching or prestraining at least a portion of one of the nonelastic webs of the elastic feature in such a way as to provide such an improved elastic feature.

These and other objects of the present invention will be more readily apparent when considered in reference to the following description and when taken in conjunction with the accompanying drawings.

### SUMMARY OF THE INVENTION

The present invention provides absorbent articles such as disposable diapers, incontinent briefs, sanitary napkins, diaper holders and the like, that have a unique elastic feature. Such absorbent articles comprise a containment assembly preferably comprising a liquid pervious topsheet, a liquid impervious backsheet, and an absorbent core positioned between the topsheet and the backsheet; and an elastic feature extending outwardly from one of the edges of the absorbent core. The elastic feature comprises an elastomeric member and a relatively nonelastic web having a portion thereof that coincides with the elastomeric member mechanically prestrained to permanently elongate that portion of the nonelastic web. The mechanical prestraining of the nonelastic web improves not only the extension of the elastomeric member but also the contraction of the elastomeric member, particularly if the elastomeric member comprises a heat shrinkable material. The elastic features of the present invention preferably comprise a portion of the topsheet, a portion of the backsheet, and the elastomeric member disposed between them wherein either the topsheet or both the topsheet and the backsheet may be mechanically prestrained. In a preferred embodiment, the elastic feature comprises an elastic waist feature that provides improved fit about the waist of the wearer during use.

The present invention also provides methods and apparatus for mechanically prestraining (mechanically stretching) at least a portion of one of the nonelastic webs of the elastic feature.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which like designations are used to designate substantially identical elements, and in which:
Figure 1 is a plan view of a disposable diaper embodiment of the present invention having portions cut-away to reveal underlying structure, the outer surface of the diaper facing the viewer;
Figure 2 is a fragmentary sectional view of the disposable diaper shown in Figure 1 taken along section line 2-2 of Figure 1;
Figure 2A is a plan view of the prestrained nonelastic web portion of the disposable diaper showing the pattern of the mechanical stretching;
Figure 3 is a fragmentary sectional view of the disposable diaper shown in Figure 1 taken along section line 3-3 of Figure 1;
Figure 4 is a simplified perspective view of an apparatus that employs a vacuum web restraint system for mechanically stretching a portion of the nonelastic web using meshing corrugated rolls;
Figure 4A is a simplified view taken along line 4A-4A in Figure 4 showing the manner in which idler rolls are used to cause the nonelastic web to wrap the lowermost corrugated rolls;
Figure 4B is a highly enlarged view taken at the inset 4B shown in Figure 4, showing the degree of meshing of the corrugated rolls with one another as the nonelastic web passes therebetween;
Figure 5 is a simplified perspective view showing an alternative web restraint system of the present invention which may be used during the incremental stretching process disclosed herein; and
Figure 5A is a highly enlarged simplified cross-sectional view taken at inset 5A shown in Figure 5 along a centerline connecting the uppermost corrugated rolls and the lowermost corrugated rolls.

### DETAILED DESCRIPTION OF THE INVENTION

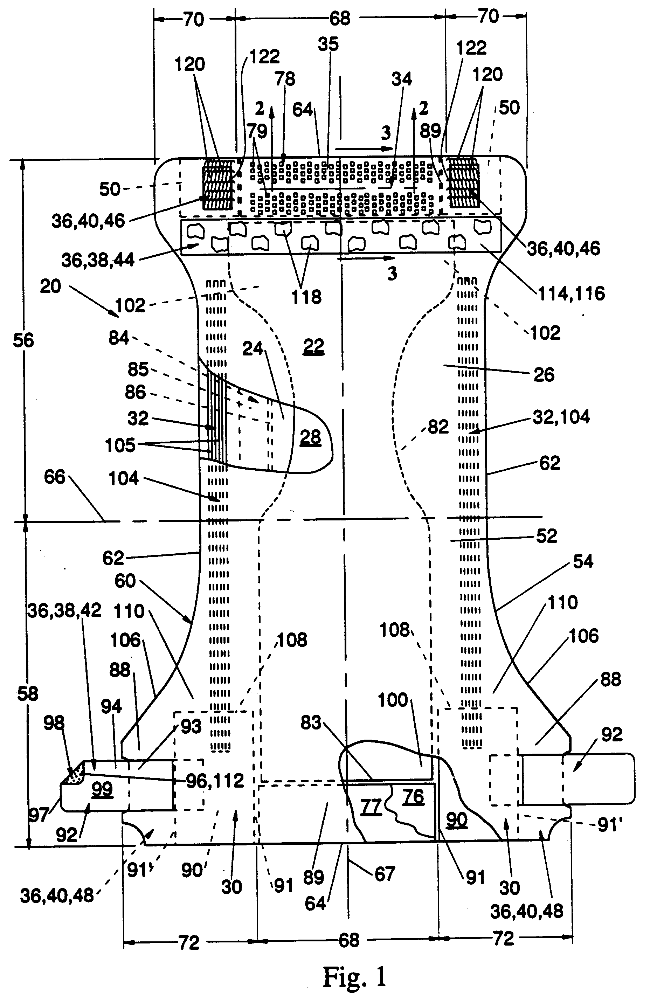

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and liner. A preferred embodiment of an absorbent article of the present invention is the unitary disposable absorbent article, diaper 20, shown in Figure 1. As used herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments such as sanitary napkins and pantiliners, and the like.

Figure 1 is a plan view of the diaper 20 of the present invention in its flat-out, uncontracted state (i.e., with elastic induced contraction pulled out except in the side panels wherein the elastic is left in its relaxed condition) with portions of the structure being cut-away to more clearly show the construction of the diaper 20 and with the portion of the diaper 20 which faces away from the wearer, the outer surface 52, facing the viewer. As shown in Figure 1, the diaper 20 comprises a containment assembly 22 preferably comprising a liquid pervious topsheet 24, a liquid impervious backsheet 26 joined with the topsheet 24, and an absorbent core 28 positioned between the topsheet 24 and the backsheet 26; elasticized side panels 30; elasticized leg cuffs 32; an elastic waist feature 34; and a closure system comprising a dual tension fastening system generally multiply designated as 36. The dual tension fastening system 36 preferably comprises a primary fastening system 38 and a waist closure system 40. The primary fastening system 38 preferably comprises a pair of securement members 42 and a landing member 44. The waist closure system 40 is shown in Figure 1 to preferably comprise a pair of first attachment components 46 and a second attachment component 48. The diaper 20 also preferably comprises a positioning patch 50 located subjacent each first attachment component 46.

The diaper 20 is shown in Figure 1 to have an outer surface 52 (facing the viewer in Figure 1), an inner surface 54 opposed to the outer surface 52, a first waist region 56, a second waist region 58 opposed to the first waist region 56, and a periphery 60 which is defined by the outer edges of the diaper 20 in which the longitudinal edges are designated 62 and the end edges are designated 64. (While the skilled artisan will recognize that a diaper is usually described in terms of having a pair of waist regions and a crotch region between the waist regions; in this application, for simplicity of terminology, the diaper 20 is described as having only waist regions, each of the waist regions including a portion of the diaper which would typically be designated as part of the crotch region). The inner surface 54 of the diaper 20 comprises that portion of the diaper 20 which is positioned adjacent to the wearer's body during use (i.e., the inner surface 54 generally is formed by at least a portion of the topsheet 24 and other components joined to the topsheet 24). The outer surface 52 comprises that portion of the diaper 20 which is positioned away from the wearer's body (i.e., the outer surface 52 generally is formed by at least a portion of the backsheet 26 and other components joined to the backsheet 26). The first waist region 56 and the second waist region 58 extend, respectively, from the end edges 64 of the periphery 60 to the lateral centerline 66 of the diaper 20. The waist regions each comprise a central region 68 and a pair of side panels which typically comprise the outer lateral portions of the waist regions. The side panels positioned in the first waist region 56 are designated 70 while the side panels in the second waist region 58 are designated 72. (In the discussion that follows, unless otherwise noted, the diaper 20 will comprise a pair of side panels in each waist region. While it is not necessary that the pairs of side panels or each side panel be identical, they are preferably mirror images one of the other.) In a preferred embodiment of the present invention, the side panels 72 positioned in the second waist region 58 are elastically extensible in the lateral direction (i.e., elasticized side panels 30). (The lateral direction (x direction or width) is defined as the direction parallel to the lateral centerline 66 of the diaper 20; the longitudinal direction (y direction or length) being defined as the direction parallel to the longitudinal centerline 67; and the axial direction (Z direction or thickness) being defined as the direction extending through the thickness of the diaper 20.)

Figure 1 shows a preferred embodiment of the diaper 20 in which the topsheet 24 and the backsheet 26 have length and width dimensions generally larger than those of the absorbent core 28. The topsheet 24 and the backsheet 26 extend beyond the edges of the absorbent core 28 to thereby form the periphery 60 of the diaper 20. The periphery 60 defines the outer perimeter or, in other words, the edges of the diaper 20. The periphery 60 comprises the longitudinal edges 62 and the end edges 64.

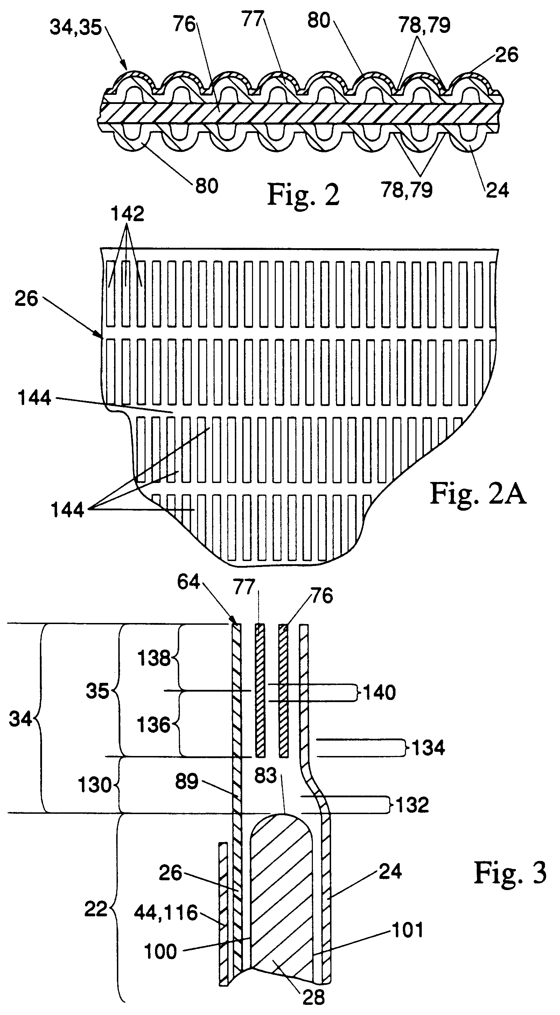

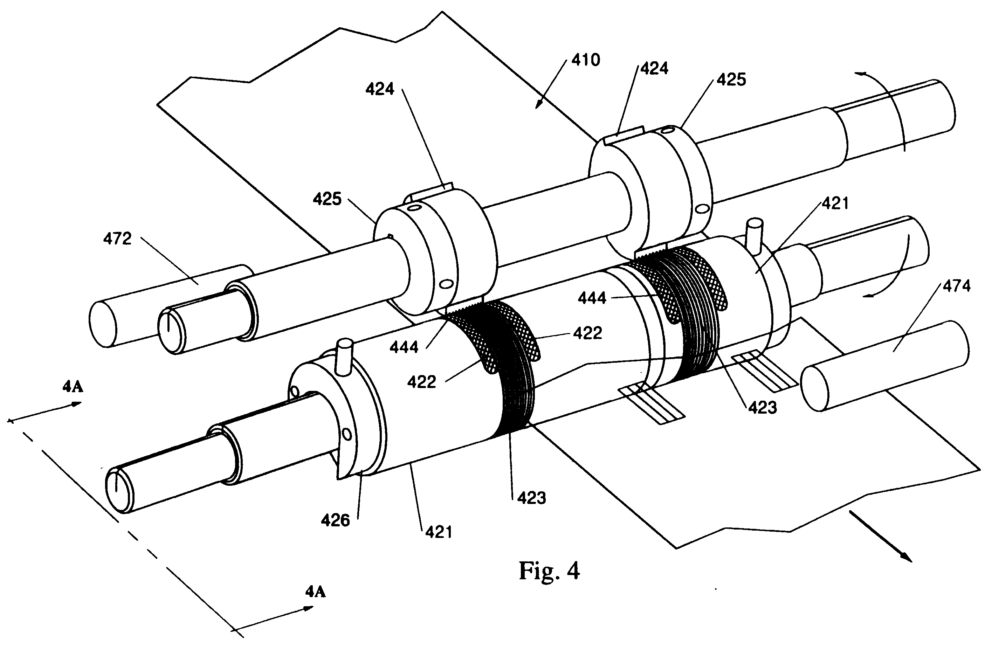

Figure 2 is a cross-sectional view of the diaper 20 taken along section line 2-2 of Figure 1 in the first waist region 56. Figure 2 shows the construction of the elasticized waistband 35 of the elastic waist feature 34. The elasticized waistband 35 is shown in Figure 2 in its contracted or relaxed condition. The elasticized waistband 35 preferably comprises a portion of the topsheet 24, a portion of the backsheet 26, and a bi-laminate material comprising an elastomeric member 76 positioned between the topsheet 24 and the backsheet 26 and a resilient member 77 positioned between the backsheet 26 and the elastomeric member 76. The elasticized waistband 35 is also provided with regions of securement 78 wherein the backsheet 26 and the topsheet 24 are joined to the bi-laminate material of the elastomeric member 76 and the resilient member 77. Since the topsheet 24 and the backsheet 26 are gathered when the bi-laminate material is In its relaxed condition, regions of differential securement are provided which form pleats 80.

Figure 3 is a fragmentary cross-sectional view of the diaper 20 taken along line 3-3 of Figure 1 and depicts a preferred elastic waist feature construction in the first waist region 56. The absorbent core 28 is generally shown in Figure 3 and shows the waist edge 83 of the absorbent core 28. The topsheet 24 and the backsheet 26 encase the absorbent core 28 and extend longitudinally outwardly beyond the waist edge 83 of the absorbent core 28 to form a waist flap 89 and the end edge 64. The elastic waist feature 34 extends longitudinally outwardly from the waist edge 83 of the absorbent core 28 in at least the central region 68 and forms at least a portion of the end edge 64. The elastic waist feature 34 comprises an interconnecting panel zone 130, a first flexural hinge zone 132 joining the interconnecting panel zone 130 with the containment assembly 22 adjacent the waist edge 83 of the absorbent core 28, an elasticized waistband 35, and a second flexural hinge zone 134 joining the elasticized waistband 35 with the interconnecting panel zone 130. As shown in Figure 3, the elasticized waistband 35 comprises a shaping panel zone 136; a waistline panel zone 138; and a predisposed, resilient, waistband flexural hinge zone 140 joining the shaping panel zone 136 and the waistline panel zone 138. As shown in Figure 3, the interconnecting panel zone 130 comprises a portion of the topsheet 24 and the backsheet 26 while the elasticized waistband 35 comprises a portion of the topsheet 24 and the backsheet 26 and the bi-laminate material of the elastomeric member 76 and the resilient member 77.

The containment assembly 22 of the diaper 20 is shown in Figure 1 as comprising the main body (chassis) of the diaper 20. The containment assembly 22 comprises at least an absorbent core 28 and preferably an outer covering layer comprising the topsheet 24 and the backsheet 26. When the absorbent article comprises a separate holder and a liner, the containment assembly 22 generally comprises the holder and the liner (i.e., the containment assembly 22 comprises one or more layers of material to define the holder while the liner comprises an absorbent composite such as a topsheet, a backsheet, and an absorbent core.) For unitary absorbent articles, the containment assembly 22 comprises the main structure of the diaper with other features added to form the composite diaper structure. Thus, the containment assembly 22 for the diaper 20 generally comprises the topsheet 24, the backsheet 26, and the absorbent core 28.

The absorbent core 28 may be any absorbent means which is generally compressible, conformable, non-irritating to the wearer's skin, and capable of absorbing and retaining liquids such as urine and other certain body exudates. As shown in Figure 1, the absorbent core 28 has a garment surface 100, a body surface 101, side edges 82, and waist edges 83.

The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which Is generally referred to as airfelt. Examples of other suitable absorbent materials include creped cellulose wadding, meltblown polymers including coform, chemically modified or cross-linked cellulosic fibers, tissue including tissue wraps and tissue laminates, absorbent foams, absorbent sponges, superabsorbent polymers, absorbent gelling materials, or any equivalent material or combinations of materials. The configuration and construction of the absorbent core may also be varied (e.g., the absorbent core may have varying caliper zones, a hydrophilic gradient, a superabsorbent gradient, or lower average density and lower average basis weight acquisition zones; or may comprise one or more layers or structures). The total absorbent capacity of the absorbent core 28 should, however, be compatible with the design loading and the intended use of the diaper 20. Further, the size and absorbent capacity of the absorbent core 28 may be varied to accommodate wearers ranging from infants through adults.

A preferred embodiment of the diaper 20 has an asymmetric, modified T-shaped, absorbent core 28 having ears 102 in the first waist region 56 but a generally rectangular shape in the second waist region 58. This configuration allows wider elasticized side panels 30 in the second waist region 58. An exemplary absorbent structure for use as the absorbent core 28 of the present invention that has achieved wide acceptance and commercial success is described in U.S. Patent 4,610,678 entitled "High-Density Absorbent Structures" issued to Weisman and Goldman on September 9, 1986. U.S. Patent 4,673,402 entitled "Absorbent Articles With Dual-Layered Cores" issued to Weisman, Houghton, and Gellert on June 16, 1987; and U.S. Patent 4,888,231 entitled "Absorbent Core Having A Dusting Layer" issued to Angstadt on December 19, 1989; also describe absorbent structures that are useful in the present invention. The absorbent core 28 may also comprise the commercially successful absorbent member described in U.S. Patent 4,834,735, entitled "High Density Absorbent Members Having Lower Density and Lower Basis Weight Acquisition Zones", issued to Alemany and Berg on May 30, 1989. The absorbent core may further comprise the dual core system containing an acquisition/distribution core of chemically stiffened fibers positioned over the absorbent storage cores as detailed above such as that system described in U.S. Patent 5,234,423.

The backsheet 26 is positioned adjacent the garment surface 100 of the absorbent core 28 and is preferably joined thereto by attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by Century Adhesives, Inc. of Columbus, Ohio and marketed as Century 5227; and by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola and Tucker on March 4, 1986. An exemplary attachment means of an open pattern network of filaments comprises several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the diaper 20 such as bedsheets and undergarments. The backsheet 26 may thus comprise a woven or nonwoven material, polymeric films such as thermoplastic films of polyethylene or polypropylene, or composite materials such as a film-coated nonwoven material. Preferably, the backsheet is a thermoplastic film having a thickness of from about 0.012 mm (0.5 mil) to about 0.051 mm (2.0 mils).

In a preferred embodiment of the present invention, at least a portion of the backsheet 26 is subjected to mechanical stretching in order to provide a "zero strain" stretch laminate that forms the elasticized side panels 30, and, alternatively if desired, to prestrain portions of the backsheet coinciding with any of the elastic features (e.g., elasticized waistband 35 or elasticized leg cuff 32). Thus, the backsheet 26 is preferably elongatable, most preferably drawable, but not elastomeric, so that the backsheet 26 will, upon mechanical stretching, be at least to a degree permanently elongated such that it will not fully return to its original undistorted configuration (i.e., the backsheet is a nonelastic web) . In preferred embodiments, the backsheet can be subjected to mechanical stretching without undue rupturing or tearing. Thus, it is preferred that the backsheet 26 have an ultimate elongation to break of at least about 400% to about 700% in the cross-machine direction as measured using a method consistent with ASTM D-638. Thus, preferred polymeric films for use as the backsheet contain a high content of linear low density polyethylene. Particularly preferred materials for the backsheet include blends comprised of about 45-90% linear low density polyethylene and about 10-55% polypropylene. Exemplary films for use as the backsheet of the present invention are manufactured by Tredegar Industries, Inc. of Terre Haute, Indiana under the designation RR8220 blend for blown films and RR5475 blend for cast films. The backsheet 26 is preferably embossed (typically, to a caliper of about 0.127 mm (5.5 mils)) and/or matte finished to provide a more clothlike appearance. Further, the backsheet 26 may permit vapors to escape from the absorbent core 28 (i.e., breathable) while still preventing exudates from passing through the backsheet 26.

The size of the backsheet 26 is dictated by the size of the absorbent core 28 and the exact diaper design selected. In a preferred embodiment, the backsheet 26 has a modified hourglass shape extending beyond the absorbent core 28 a minimum distance of at least about 1.3 cm to about 2.5 cm (about 0.5 to about 1.0 inch) around the entire diaper periphery 60. Preferably, the backsheet 26 is much wider than the absorbent core 28 in the second waist region 58 so that the side panels 72 in the second waist region 58 are generally wider in the lateral direction than the side panels 70 in the first waist region 56.

The topsheet 24 is positioned adjacent the body surface 101 of the absorbent core 28 and is preferably joined thereto and to the backsheet 26 by attachment means (not shown) such as those well known in the art. Suitable attachment means are described with respect to joining the backsheet 26 to the absorbent core 28. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. In a preferred embodiment of the present invention, the topsheet 24 and the backsheet 26 are joined directly to each other in the diaper periphery 60 and are indirectly joined together by directly joining them to the absorbent core 28 by the attachment means (not shown).

The topsheet 24 is compliant, soft feeling, and non-irritating to the wearer's skin. Further, the topsheet 24 is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Preferably, the topsheet 24 is made of a hydrophobic material to isolate the wearer's skin from liquids contained in the absorbent core 28.

In a preferred embodiment of the present invention, at least a portion of the topsheet 24 is subjected to mechanical stretching in order to provide a "zero strain" stretch laminate that forms the elasticized side panels 30, and, alternatively if desired, to prestrain portions of the topsheet coinciding with any of the elastic features. Thus, the topsheet 24 is preferably elongatable, most preferably drawable, but not elastomeric, so that the topsheet 24 will, upon mechanical stretching, be at least to a degree permanently elongated such that it will not fully return to its original configuration (i.e., the topsheet is a nonelastic web). In preferred embodiments, the topsheet 24 can be subjected to mechanical stretching without undue rupturing or tearing of the topsheet. Thus, it is preferred that the topsheet 24 have a low yield strength in the direction of stretching (e.g., low cross-machine direction (lateral direction) yield strength for use in the elasticized side panels and/or the elasticized waistband).

There are a number of manufacturing techniques which may be used to manufacture the topsheet 24. For example, the topsheet 24 may be a nonwoven web of fibers. When the topsheet comprises a nonwoven web, the web may be spunbonded, carded, wet-laid, meltblown, hydroentangled, combinations of the above, or the like. A preferred topsheet is carded and thermally bonded by means well known to those skilled in the fabrics art. A preferred topsheet comprises staple length polypropylene fibers having a denier of about 2.2. As used herein, the term "staple length fibers" refers to those fibers having a length of at least about 15.9 mm (0.625 inches). Preferably, the topsheet has a basis weight from about 18 to about 25 grams per square meter. A suitable topsheet is manufactured by Veratec, Inc., a Division of International Paper Company, of Walpole, Massachusetts under the designation P-8.

The diaper 20 preferably further comprises an elastic leg feature, elasticized leg cuffs 32, for providing improved containment of liquids and other body exudates. Each elasticized leg cuff 32 may comprise several different embodiments for reducing the leakage of body exudates in the leg regions. (The leg cuff can be and is sometimes also referred to as leg bands, side flaps, barrier cuffs, or elastic cuffs.) U.S. Patent 3,860,003 entitled "Contractable Side Portions For a Disposable Diaper" issued to Buell on January 14, 1975, describes a disposable diaper which provides a contractible leg opening having a side flap and one or more elastic members to provide an elasticized leg cuff (gasketing cuff) extending laterally outwardly from the side edge of the absorbent core. U.S. Patent 4,909,803 entitled "Disposable Absorbent Article Having Elasticized Flaps" issued to Aziz and Blaney on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987, describes a disposable diaper having dual cuffs including a gasketing cuff and a barrier cuff. U.S. Patent 4,704,115 entitled "Disposable Waist Containment Garment" issued to Buell on November 3, 1987, discloses a disposable diaper or incontinent garment having side-edge-leakage-guard gutters configured to contain free liquids within the garment. While each elasticized leg cuff 32 may be configured so as to be similar to any of the leg bands, side flaps, barrier cuffs, or elastic cuffs described above, it is preferred that each elasticized leg cuff 32 comprise at least an inner barrier cuff 84 comprising a barrier flap 85 and a spacing elastic member 86 such as described in the above-referenced U.S. Patent 4,909,803. In a preferred embodiment, the elasticized leg cuff 32 additionally comprises an elastic gasketing cuff 104 with one or more elastic strands 105, positioned outboard of the barrier cuff 84 such as described in the above-referenced U.S. Patent 4,695,278. In an alternative embodiment of the present invention and as described hereinafter, the nonelastic web components of the elasticized leg cuffs, typically either or both of the topsheet and the backsheet, may be mechanically prestrained to enhance the extension and contraction of the elasticized leg cuff.

The diaper 20 further comprises an elastic waist feature 34 that provides improved fit and containment. The elastic waist feature 34 at least extends longitudinally outwardly from at least one of the waist edges 83 of the absorbent core 28 in at least the central region 68 and generally forms at least a portion of the end edge 64 of the diaper 20. Thus, the elastic waist feature 34 comprises that portion of the diaper at least extending from the waist edge 83 of the absorbent core 28 to the end edge 64 of the diaper 20 and is intended to be placed adjacent the wearer's waist. Disposable diapers are generally constructed so as to have two elastic waist features, one positioned in the first waist region and one positioned in the second waist region. While a disposable diaper of the present invention can be constructed with a single elastic waist feature encircling the wearer, the discussion regarding the elastic waist feature will focus on diapers having a pair of elastic waist features, at least one, and preferably both, being constructed according to the present invention. Further, while the elastic waist feature or any of its constituent elements can comprise a separate element affixed to the containment assembly 22 of the diaper 20, the elastic waist feature 34 will be described with respect to a preferred embodiment in which the elastic waist feature 34 is constructed as an extension of other elements of the diaper such as the backsheet 26 or the topsheet 24, preferably both the backsheet 26 and the topsheet 24.

While the elastic waist feature 34 may comprise a number of configurations (one such configuration being shown in the previous referenced U.S. 4,515,595 of Kievit), the elastic waist feature 34 preferably comprises, as shown in figure 3, an interconnecting panel zone 130, a first flexural hinge zone 132 joining the interconnecting panel zone 130 with the containment assembly 22 adjacent the waist edge 83 of the absorbent core 28, an elasticized waistband 35, and a second flexural hinge zone 134 joining the elasticized waistband 35 with the interconnecting panel zone 130. As shown in Figure 3, the elasticized waistband 35 comprises a shaping panel zone 136; a waistline panel zone 138; and a predisposed, resilient, waistband flexural hinge zone 140 joining the shaping panel zone 136 and the waistline panel zone 138. As used herein, the term "zone" is used to denote an area or element of the elastic waist feature 34. While a zone of the elastic waist feature 34 may be a distinct area or element; typically, a zone of the elastic waist feature will overlap somewhat with an adjacent zone(s). (For illustration purposes, the zones are delineated with brackets in Figure 3.) A more detailed description of the elastic waist feature shown in Figure 3 is found in U.S. Patent 5,151,092.

The elasticized waistband 35 may be constructed in a number of different configurations. In a preferred embodiment as shown in Figure 2, the elasticized waistband 35 preferably is constructed from four materials laminated together. The elasticized waistband 35 preferably comprises a portion of the topsheet 24; a portion of the backsheet 26; an elastomeric member 76; and a resilient member 77. The elastomeric member 76 and the resilient member 77 are preferably joined together, preferably by dynamic mechanical bonds, to form an elastic laminate prior to being combined with the topsheet 24 and the backsheet 26. This bi-laminate is preferably positioned between the topsheet 24 and the backsheet 26 with the resilient member 77 disposed toward the backsheet 26 and the elastomeric member 76 disposed toward the topsheet 24. Thus, the elastomeric member 76 is preferably positioned between the topsheet 24 and the backsheet 26 with the resilient member 77 preferably being positioned between the backsheet 26 and the elastomeric member 76.

The elastomeric member 76 is operatively associated with the elasticized waistband 35, preferably between the topsheet 24 and the backsheet 26, so that the elastomeric member 76 allows the elasticized waistband 35 to be elastically extensible in the lateral direction (i.e., laterally elastically extensible), and so that it can contractively return to its substantially unrestrained configuration. The elastomeric member 76 can be operatively associated in the elasticized waistband 35 in a number of different ways. As an example, the elastomeric member may be operatively associated in an elastically contractible condition so that the elastomeric member gathers or contracts the elasticized waistband. (A more detailed description of the manner in which elastomeric materials may be secured in an absorbent article in an elastically contractible condition can be found in U.S. Patent 3,860,003 issued to Buell on January 14, 1975, and in U.S. Patent 4,081,301 issued to Buell on March 28, 1978.) For example, the elastomeric members 76 can be contractibly affixed in the elasticized waistband 35 by laterally extending the elastomeric member 76, affixing the elastomeric member 76 to either or both the topsheet 24 and the backsheet 26, and allowing the elastomeric member 76 to assume its relaxed or contracted orientation.

Alternatively, the elastomeric member 76 can be operatively associated in the elasticized waistband 35 by securing the elastomeric member 76 to the topsheet 24, the backsheet 26, or both while the elastomeric member 76 is in a substantially untensioned condition, at least a portion of the laminate containing the elastomeric member 76 then being subjected to mechanical stretching sufficient to permanently elongate the topsheet 24 and the backsheet 26 components of the laminate, and then the composite or elastomeric laminate is returned to its substantially untensioned condition. The elasticized waistband 35 is thus formed into a "zero strain" stretch laminate. The elastomeric laminate may alternatively be operatively associated in a tensioned condition and subjected to mechanical stretching to form a mechanically stretched, pretensioned, stretch laminate.)

In an especially preferred embodiment as shown in Figure 2, the elastomeric member 76 can be operatively associated in an uncontracted state and then treated to contract. In this embodiment, the elastomeric member 76 can be formed from materials which contract unidirectionally and become elastic following specific treatment such as heating. Examples of such materials are disclosed in U.S. Patent 3,819,401 issued to Massengale, et al. on June 25, 1974 and in U.S. Patent 3,912,565 issued to Koch, et al. on October 14, 1975. A more detailed description of a manner for using a heat-shrinkable elastomeric member is described in U.S. Patent 4,515,595 issued to Kievit and Osterhage on May 7, 1985. Typically, the topsheet 24, the backsheet 26, the elastomeric member 76, and any other components are secured together while in an uncontracted condition. The laminate is then heated (as with heated air) and the elastomeric member 76 is allowed to return to its relaxed or contracted orientation.

The elastomeric members useful in the present invention may take on a number of different sizes, shapes, configurations, and materials. For example, the elasticized waistband may be formed from one or a plurality of elastomeric members operatively associated between the topsheet and the backsheet; the elastomeric member may have varying widths and lengths; or the elastomeric member may comprise relatively narrow strands of elastomeric material or a larger area patch of elastomeric material. One elastomeric material which had been found to be suitable for use as the elastomeric member is an elastomeric foam such as the polyurethane foam such as is available from Bridgestone of Yokahama, Japan and designated Bridgestone SG Polyurethane Foam. Other suitable elastomeric materials for use as the elastomeric member include "live" synthetic or natural rubber, elastomeric films (including heat-shrinkable elastomeric films), formed elastomeric scrim, or the like. In an especially preferred embodiment as is shown in Figure 2, the elastomeric member 76 comprises a heat-shrinkable elastomeric film such as marketed by Exxon Chemical Company of Florham Park, New Jersey.

The resilient member 77 is a layer or layers that provides enhanced shape recovery and bending stiffness to the elasticized waistband 35. The resilient member 77 provides compression/buckling resistance In the longitudinal direction (machine direction) so that the waistband flexural hinge zone 140 will be resilient so as to provide a restoring force/moment. The resilient member 77 also has a relatively high caliper to provide a Z-direction bulk so as to somewhat fill the pleats 80 or rugosities of the elasticized waistband 35 so as to optimize its resiliency. The resilient member 77 is also preferably hydrophobic to prevent wicking of liquids out of the elasticized waistband 35. In an especially preferred embodiment, the resilient member 77 comprises a nonwoven material in which the fibers are oriented in the machine direction (longitudinal direction of the diaper) to reduce cross machine direction (lateral) gather resistance so that the heat shrinkable elastomeric member can readily contract and to enhance the compression resistance about a defined axes of bending, the waistband flexural hinge zone 140.

While the resilient member 77 is preferably positioned between the elastomeric member 76 and the backsheet 26 as shown In Figure 3, the resilient member 77 may alternatively be positioned between the topsheet 24 and the elastomeric member 76, on the outside of the backsheet 26, on the outside of the topsheet 24, or in a number of other configurations. The resilient member 77 is preferably positioned between the backsheet 26 and the elastomeric member 76 to provide greater compression/buckling resistance on the backsheet side of the elasticized waistband 35 since it will fill the rugosities or pleats 80 in the longitudinal axis between the bond sites, for providing bending resistance about the lateral axis of the elasticized waistband, and to optimize and reduce wicking in the laminate material forming the elasticized waistband.

The resilient member 77 may take on a number of different sizes, shapes, configurations, and materials. For example, the resilient member may be formed from one or a plurality of resilient members; the resilient member may have varying widths, lengths, thickness, and shapes. The resilient member 77 preferably comprises a separate piece of material positioned in the elasticized waistband. Alternatively, the resilient member may comprise a portion of any or all of the materials making up the elasticized waistband 35, including the elastomeric member 76, the topsheet 24, or the backsheet 26. In a particular alternative embodiment, the resilient member 77 comprises the same piece as the elastomeric member 76, the elastomeric member 76 comprising a relatively thick elastomeric foam.

Suitable materials for use as the resilient member 77 of the elasticized waistband 35 of the present invention include woven webs; nonwoven webs; foams; laminate materials including film laminates of nonwoven laminates of two or more nonwoven layers; scrims; corrugated materials that provide stiffness in at least one direction; and any combination of the above materials or other materials as are known in the art.

Particularly preferred materials for the resilient member 77 comprise nonwoven webs that are hydrophobic and that have a drapability (as measured using ASTM B1388-64) of at least about 4 cm in the cross machine direction and at least about 12 cm in the machine direction. An exemplary material comprises a hydrophobic, nonwoven carded web having a basis weight in the range of from about 20-45 grams per square yard. The fibers are oriented in the machine direction to provide enhanced buckling/compression resistance in this direction. The nonwoven web is comprised of 3 denier bicomponent fibers of polyester core material and copolyolefin sheathing material, such fibers being available from Hoecht as CELBOND stable fibers; or of a polypropylene core material and polyethylene sheathing material, such fibers being available from Danaklon or Hercules. After carding the nonwoven web to orient the fibers in the machine direction, the nonwoven web is put through an air-thru bonding process to provide bulk (loft or thickness) to the resilient member to enhance its resiliency. (Examples of high loft nonwoven webs of bicomponent thermoplastic resin fibers that are air-thru bonded are disclosed in U.S. Patent 4,883,707 issued to Newkirk on November 28, 1989.) Exemplary air-thru bonded nonwoven carded webs of bicomponent thermoplastic fibers are available from Fiberweb North America of Greenville, North Carolina or from Veratec Corporation of Walpole, Massachusetts.

As described herein, the mechanically prestrained nonelastic web elastic features of the present invention are particularly suitable for use as an elastic waist feature. An elastic waist feature preferably comprises a heat shrinkable elastomeric member which performance is particularly enhanced by the present invention. However, the present invention finds equal facility in elasticized leg cuffs, elasticized side panels, an overall elasticized diaper, or any other elastic feature on an absorbent article comprising at least an elastomeric member and a nonelastic member.

In the present invention, a portion of at least one of the nonelastic webs forming the elastic feature is "prestrained" or "mechanically prestrained" (i.e., subjected to some degree of localized pattern mechanical stretching to permanently elongate portions of the nonelastic web forming the elastic feature prior to its incorporation into the elastic feature). A prestrained nonelastic web improves not only the extension of the elastomeric member but also the heat-shrink contraction of the preferred elastomeric members 76.

As used herein, the term "nonelastic web" means a web that is elongatable, preferably drawable, but not elastomeric so that it will, upon mechanically stretching, be at least to a degree permanently elongated such that the web will not fully return to its original configuration. In a preferred embodiment of the present invention, the elastic feature typically comprises a segment of the topsheet which extends beyond the edge of the absorbent core, a segment of the backsheet which extends beyond the edge of the absorbent core, and an elastomeric member disposed between the topsheet and the backsheet. Thus, the topsheet and the backsheet comprise nonelastic webs forming the elastic feature. Alternatively or in addition to the topsheet and the backsheet, the elastic feature may comprise any of a number of nonelastic webs forming the elastic feature (e.g., the resilient member as shown in the preferred elasticized waistbands of the present invention).

Since the elastic features of the present invention comprises a portion of the topsheet and a portion of the backsheet, the topsheet, the backsheet, or the topsheet individually, may be mechanically prestrained prior to their incorporation into the elastic features. Since the backsheet typically comprises a polymeric thermoplastic film, it is the backsheet web that more typically inhibits the expansion and contraction of the elastic features. It has also been found that mechanically prestraining the topsheet can provide enhanced performance of the elastic features. Thus, in an alternatively preferred embodiment, at least the portion of the topsheet coinciding with at least a portion of the elastomeric member is mechanically prestrained. Further, mechanical straining of both the topsheet and the backsheet individually will also enhance the performance of the elastic features.

While the elastic features have been described herein as having at least the portion of the nonelastic web coinciding with at least a portion of the elastomeric member mechanically prestrained, the present invention also contemplates embodiments wherein the entire portion of the nonelastic web that coincides with all of the elastomeric member is mechanically prestrained, wherein a large area of the nonelastic web extending beyond the area of the elastomeric member is mechanically prestrained, or wherein the entire nonelastic web itself Is mechanically prestrained. It is, however, important that at least a portion of the nonelastic web that coincides with at least a portion of the elastomeric member be mechanically prestrained to provide the benefits described herein.

In a preferred embodiment as shown in Figure 2A, the prestrained nonelastic web assumes a pattern of ribs 142 (strained portions) and unstrained gaps 144 between each rib 142 and between each row of ribs. This pattern is determined by the method and apparatus used to prestrain the nonelastic web. In this preferred embodiment, the ribs are 0.25 inch (about 6 mm) long, 0.030 inch (about .75 mm) wide, have a depth of 0.125 inch (about 3 mm), with a spacing between each rib (gap) of 0.100 inch (about 2.5 mm). The area of the nonelastic web that is prestrained, when an elasticized waistband 35 is formed, is preferably about 5.5 inches (about 140 mm) wide by about 1 inch (about 25 mm) in length.

The nonelastic web can be prestrained by directing it through an incremental mechanical stretching system such as is described hereinafter prior to combining the nonelastic web into the finished diaper product. The corrugated or grooved segments contained on the rolls are interrupted to bring about a pattern of ribs in the machine direction and the cross-machine direction. The registration of the nonelastic web is such that the portion of the nonelastic web to be prestrained substantially coincides with the corrugated or grooved segments contained on the uppermost corrugated rolls as the nonelastic web passes between the segments of the uppermost corrugated rolls and the corrugated or grooved lowermost corrugated rolls. The nonelastic web can alternatively be prestrained by using deep embossing techniques as are known in the art.

Particularly preferred methods and apparatus used for mechanically prestraining a web of material use meshing corrugated rolls to mechanically stretch the web. A discussion of suitable apparatus and methods for mechanically stretching portions of a diaper is contained in U.S. Patent 4,107,364 issued to Sisson on August 15, 1978 and U.S. Patent 4,834,741 issued to Sabee on May 30, 1989. Particularly preferred apparatus and methods are disclosed in U.S. Patent 5,167,897; U.S. Patent 5,156,793; and U.S. Patent 5,143,679.

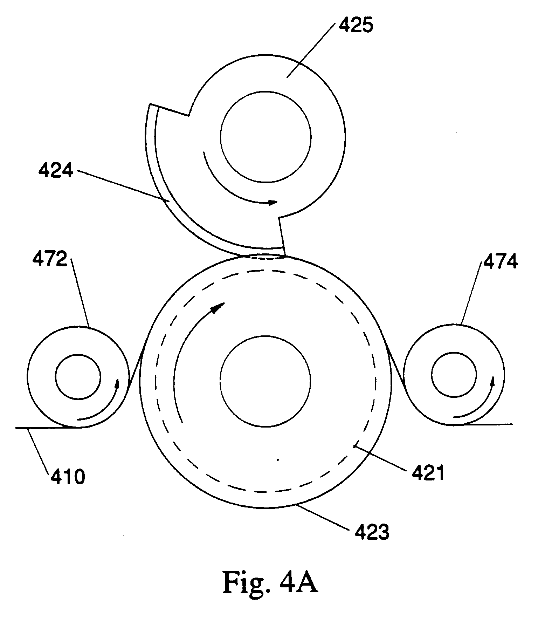

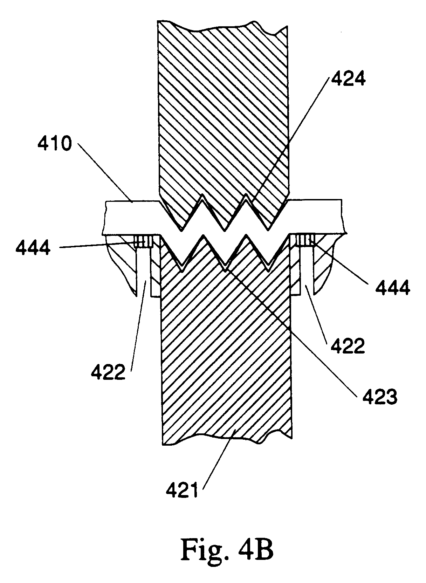

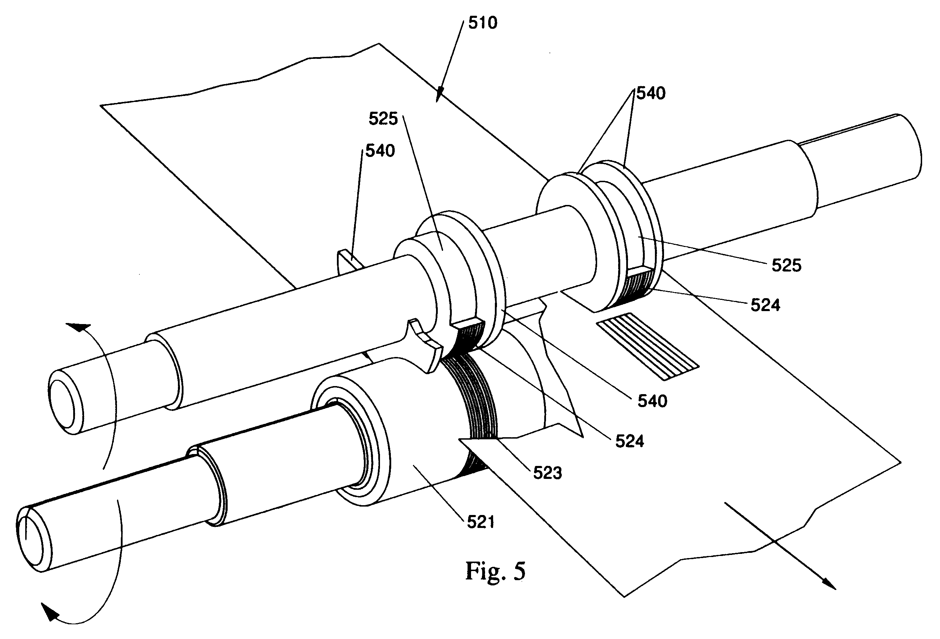

Details of a particularly preferred incremental stretching system which can be employed In mechanically prestraining a nonelastic web are set forth In Figure 4. Since the backsheet web, the topsheet web, or any other inelastic web forming a portion of the elastic feature may be prestrained, the incremental stretching system will be generally described in relation to directing a nonelastic web 410 therethrough.

Referring to Figure 4, the timing of the nonelastic web 410 is such that the portion of the web which will coincide with the elastomeric member substantially coincides with the corrugated or grooved segments 424 contained on the uppermost corrugated rolls 425 as the nonelastic web 410 passes between the segments 424 of the uppermost corrugated rolls 425 and the continuously corrugated or grooved lowermost corrugated rolls 421.

While the exact configuration, spacing and depth of the complementary grooves on the uppermost and lowermost corrugated rolls will vary, a peak-to-peak groove pitch of approximately 0.150 inches, an included angle of approximately 12 degrees as measured at the peak, and a peak-to-valley groove depth of approximately 0.300 inches have been employed In a particularly preferred embodiment of the present invention. The exterior peak of each corrugation on the aforementioned corrugated rolls typically exhibits a radius of approximately 0.010 inches, while the internal groove formed between adjacent corrugations typically exhibits a radius of approximately 0.040 inches. When the corrugated rolls are adjusted so that their opposing peaks overlap one another to a depth between about 0.150 and about 0.175 inches, good characteristics have been produced. (In alternative embodiments, especially for use in prestraining the topsheet, the corrugated rolls may have a peak-to-peak groove pitch of 0.060 inches and a peak-to-valley groove depth of 0.060 inches. Further, a rubber back up roll may be substituted for one of the grooved rolls.)

The degree of overlap of the opposing peaks on the aforementioned corrugated rolls may of course be adjusted, as desired, to produce more or less strain in the nonelastic web. For the aforementioned roll geometry and web construction, peak-to-peak overlap depths ranging from as little as about 0.050 inches to as much as about 0.225 inches are feasible.

As can be seen from Figure 4A, the nonelastic web 410 is caused by the idler rolls 472, 474 to wrap the lowermost corrugated rolls 421 sufficiently to cover the active vacuum ports 422 (shown in Figure 4) located immediately adjacent each continuous set of grooves 423 on the lowermost rolls 421. The vacuum ports 422, which are positioned so as to substantially coincide with the grooved segments 424 on the uppermost corrugated rolls 425, are internally connected through the rolls 421 to a pair of vacuum manifolds 426 which exert suction against the nonelastic web 410 as the web is acted upon by the grooved segments 424 of the uppermost corrugated rolls 425.

The vacuum ports 422 on the lowermost rolls 421 are preferably covered by a porous material, such as 0.090 inch mesh honeycomb 444, to provide support to the portions of the nonelastic web 410 acted upon by the vacuum and to provide a good gripping surface against the web so as to substantially prevent lateral slippage or movement of the web across the honeycomb surface whenever the web is acted upon by the vacuum.

Under optimum circumstances, the maximum degree of incremental stretching which can be imparted to the web is determined by the depth of engagement between the grooves on segments 424 of the uppermost corrugated rolls 425 and the continuous grooves 423 on the lowermost corrugated rolls 421. However, it has been discovered that unless the web is substantially prevented from slipping or contracting in a direction substantially parallel to the direction of web stretching as it passes between the meshing corrugated rolls, the optimum degree of incremental stretching is not realized. Therefore, in its most preferred form, the incremental web stretching operation is carried out while the web is subjected to restraint, as generally shown in the cross-section of Figure 4B, to substantially prevent the web from slipping or contracting in a direction parallel to the desired direction of stretching as it passes between the sets of sequentially positioned meshing corrugated rolls.

Because the portions of the nonelastic web 410 are laterally restrained throughout the sequential web stretching operation, all portions of the web located intermediate the points of restraint are subject to substantially uniform incremental stretching as the web passes between the continuous grooves 423 on the lowermost corrugated rolls 421 and the meshing portions of the grooved segments 424 on the uppermost corrugated rolls 425.

This maximizes the effectiveness of the incremental web stretching operation by forcing the elongatable web to undergo the fullest possible degree of elongation during the stretching operation.

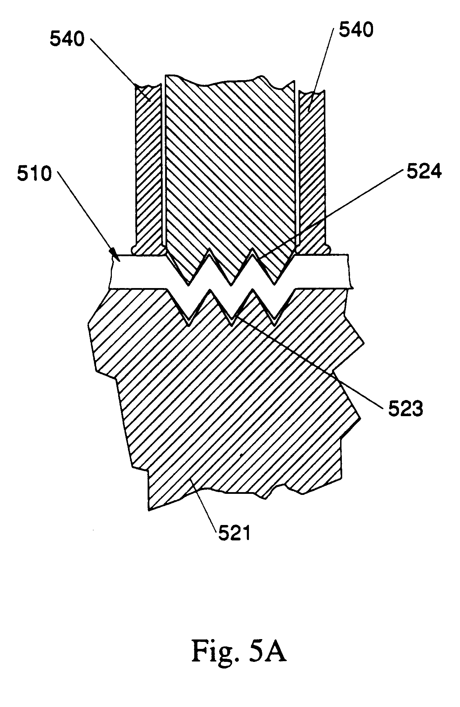

Figure 5 discloses an alternative incremental web stretching system which can be employed. In the incremental web stretching system shown in Figure 5, a pair of resiliently compressible disks 540 are mounted adjacent each side of the grooved segments 524 of the uppermost corrugated rolls 525. The compressible disks 540 are of a large enough diameter that they tightly grip the nonelastic web 510 and hold it securely against the coinciding non-grooved portions of the lowermost corrugated rolls 521 as generally shown in the cross-section of Figure 5A. Like the vacuum ports and the porous honeycomb material in the embodiment of Figure 4, the clamping effect created by the compressible disks 540 and the coinciding non-grooved portions of the lowermost rolls 521 substantially prevents the portion of the nonelastic web 510 from contracting in a direction parallel to the direction of stretching as the web passes between the meshing corrugated rolls.

As will be appreciated by those skilled in the art, the foregoing restraint methods may be employed either individually or in combination with one another to produce the benefits herein described.

From the description contained herein, it is clear that the improved method and apparatus may be employed to advantage to produce a wide range of absorbent articles either comprised entirely of or including one or more discrete, prestrained web elastic features.

It is also recognized that while a pair of meshing corrugated rolls having their corrugations aligned substantially parallel to one another are disclosed in the accompanying drawings, the present invention may be practiced with equal facility employing pairs of corrugated rolls wherein the corrugations are not all oriented parallel to one another. Furthermore, the corrugations on such pairs of corrugated rolls need not necessarily be aligned parallel to either the machine or the cross-machine direction. For example, if a curvilinear waistband or legband portion is desired in a single use diaper constructed using the technology herein disclosed, the meshing teeth on the pairs of corrugated rolls employed to incrementally stretch the web may be arrayed in the desired curvilinear configuration to produce elasticity along the desired curvilinear contour rather than in a straight line.

It is further recognized that while the preferred processes herein disclosed employ meshing cylindrical corrugated rolls, the web restraint principles may also be carried out utilizing an intermittent stamping operation employing meshing platens to incrementally stretch the web in question. In the latter instance, the only requirement is that the portions of the web to be incrementally stretched be adequately restrained by suitable vacuum or clamping means before the meshing platens are able to exert enough force on the web to cause slippage or contraction in a direction parallel to the direction of stretching.

In a preferred method for making the diapers of the present invention, after the web has been prestrained, and after the web has been removed from the corrugated combining rolls, a continuous spray glue is applied to the web. The elastic laminate comprising the resilient member and the heat-shrinkable elastomeric member is dynamically mechanically bonded with the other nonelastic web. The resulting laminate is then applied to the prestrained web of the diaper and dynamically mechanically bonded together to form the elasticized waistband 35. This diaper web is then passed to a heat shrink apparatus to contract the heat shrinkable elastomeric member.

The elasticized waistband 35 further comprises transverse regions of securement 78 shown in a generalized representation in Figure 1 and in Figure 2. A more detailed description of the transverse regions of securement and alternative configurations for them are found in U.S. Patent 4,515,595 issued to Kievit and Osterhage on May 7, 1985. The transverse regions of securement 78 extend essentially across the full width of the elasticized waistband 35, particularly the elastomeric member 76. The term "essentially across" is used in this context to indicate that the transverse regions of securement need not extend absolutely across the entire width of the elastomeric member 76 so long as they extend sufficiently far across the width to provide the function discussed hereinafter. As illustrated, the transverse regions of securement 78 are shown to be disposed at essentially a right angle to the lateral centerline 66 and to the lateral extent of the elasticized waistband 35. This is the preferred orientation.

In Figure 1, the transverse regions of securement 78 are shown as discrete, spaced, securement zones 79 effectively attaching the webs of material forming the elasticized waistband 35 (the topsheet 24, the backsheet 26, the resilient member 77, and the elastomeric member 76 in a preferred embodiment) together. While the shape of the discrete securement zones may vary, the discrete securement zones 79 are preferably circular, elliptical, oval, rectangular, or square shaped. The discrete securement zones 79 are preferably regularly spaced In a pattern (except where the waistband flexural hinge zone 140 is formed), although they can be nonuniformly spaced. The precise means for providing the securement zones 79 can be readily selected by those skilled in the art. Examples of such attachment means include adhesive attachment, heat sealing, solvent sealing, autogeneous bonding, dynamic mechanical bonding, ultrasonic welding, and the like. Preferably, the transverse regions of securement 78 comprise oval (rounded rectangular) shaped discrete securement zones 79, preferably dynamic mechanical bonds, such as described in U.S. Patent 4,919,738 entitled "Dynamic Mechanical Bonding Method And Apparatus" which issued to Ball, Goulait & Zorb on April 24, 1990, disposed in rows and columns with one column missing or irregularly spaced to form the waistband flexural hinge zone 140. The securement zones 79 are preferably from about 2.0 mm (about 0.078 in) by about 1.3 mm (about 0.52 in) and are preferably spaced from about 7.0 mm (0.275 in) to about 8.9 mm (0.375 in) from center to center in the transverse direction and from about 1.9 mm (0.375 in) to about 3.8 mm (0.15 in) from center to center in the longitudinal direction. (One row of bond sites are eliminated in each column such that there is a longitudinal gap of about 1.0 mm (about 0.040 in) to about 3.8 mm (0.150 in) from center to center to form the waistband flexural hinge zone 140.) In the most preferred embodiment, the securement zones 79 are spaced about 8.3 mm (0.325 in) from center to center In the transverse direction and about 2.8 mm (0.112 in) from center to center in the longitudinal direction with an offset gap spacing of about 0.71 mm (0.028 in) from center to center between adjacent rows in the longitudinal direction.

As illustrated in Figure 2, the discrete securement zones 79 on either side of the elastomeric member 76 are in register (i.e., they are coextensive). This is a preferred orientation, but the discrete securement zones 79 of the topsheet 24 to the elastic laminate material can be offset from the adjacent discrete securement zones 79 of the backsheet 26 to the elastic laminate material.

The diaper 20 is also preferably provided with a closure system (tensioning means) for dynamically creating/maintaining lateral tension through the elasticized waistband 35. The lateral tension dynamically created and maintained by the closure system "activates" the stretch of the elasticized waistband 35 thereby allowing it to more dynamically expand and contract with the motions of the wearer. Gapping of the elasticized waistband is also reduced by the activated stretch since It is held in tension to snugly fit against the wearer's waist both when the diaper is initially fitted to the wearer and during use. Further, rollover of the elasticized waistband is reduced by the tension created/maintained by the closure system. Thus, the closure system improves the fit and containment characteristics of the diaper.

While the closure system may take on a number of configurations such as adhesive tape tabs, mechanical closure tape tabs, fixed position fasteners, or any other means for tensioning the elasticized waistband as are known in the art; as shown in Figure 1, the closure system preferably comprises a waist closure system 40 comprising at least one, typically a pair of, first attachment components 46 and at least one second attachment component 48. More preferably, the closure system additionally comprises a primary fastening system 38 such that the diaper 20 has a dual tension fastening system 36. Preferred embodiments of a diaper having a dual tension fastening system are described in EUROPEAN PUBLICATION 0 588 916 PUBLISHED December 23, 1992; and the above referenced U.S. Patent 5,151,092.

The diaper 20 additionally comprises a positioning patch 50 located subjacent the first attachment component 46. The positioning patch 50 raises the first attachment component 46 in the Z direction (thickness) to allow the first attachment component 46 to come in better contact with the second attachment component 48 and allow the waist closure system to more easily be closed (with less effort). Thus, the waist closure system 40 is more effectively passively activated. The positioning patch 50 also provides a zone of increased flexural stiffness that reduces the tendency of the flexible ear flaps 88 to fold over onto the first attachment component(s) 46 thereby occluding the hooks from being secured during diaper application. Thus, the positioning patch 50 can comprise any element that provides a Z direction build up to the first attachment components 46. As shown in Figure 1, the positioning patches 50 each comprise a rectangular-shaped piece of material positioned subjacent the first attachment component 46. While the positioning patches 50 may be positioned directly subjacent the first attachment components 46, the positioning patches 50 are preferably positioned between the topsheet 24 and the backsheet 26. In order to provide a flexurally stiff circumference about the waist of the wearer, the lateral edges of the positioning patches can be abutted to or slightly overlapped with the side edges 75 of the elastic waistband member 76. The positioning patches 50 preferably comprise a 38 mm wide by 32 mm long patch of elastomeric foam. More preferably, during manufacture of the diaper, the positioning patches 50 are formed of the same material as the elastic side panel member 90 with the elastic side panel member 90 of one diaper and the positioning patch 50 of the adjacent diaper being formed from the same segment of material that is then cut after the diaper Is completed. Thus, the positioning patch 50 extends from the end edge 64 of the diaper 20 inward toward the center of the diaper 20.

In a preferred embodiment, the diaper also comprises elasticized side panels 30 disposed in the second waist region 58. (As used herein, the term "disposed" is used to mean that an element(s) of the diaper is formed (joined and positioned) in a particular place or position as a unitary structure with other elements of the diaper or as a separate element joined to another element of the diaper.) The elasticized side panels 30 provide an elastically extensible feature that provides a more comfortable and contouring fit by initially conformably fitting the diaper to the wearer and sustaining this fit throughout the time of wear well past when the diaper has been loaded with exudates since the elasticized side panels allow the sides of the diaper to expand and contract. Further, the elasticized side panels 30 develop and maintain wearing forces (tensions) that enhance the tensions developed and maintained by both the primary fastening system 38 and the waist closure system 40 to maintain the diaper 20 on the wearer and enhance the waist fit. The elasticized side panels 30 especially assist in initially pretensioning the elasticized waistband 35 since the diaperer typically stretches the elasticized side panels 30 when applying the diaper 20 on the wearer so that when the elasticized side panels 30 contract, tension is transmitted from the elasticized side panels 30 through the waist closure system 40 into the elasticized waistband 34. The elasticized side panels 30 further provide more effective application of the diaper 20 since even if the diaperer pulls one elasticized side panel 30 farther than the other during application (asymmetrically), the diaper 20 will "self-adjust" during wear. While the diaper 20 of the present invention preferably has the elasticized side panels 30 disposed in the second waist region 58; alternatively, the diaper 20 may be provided with elasticized side panels 30 disposed in the first waist region 56 or in both the first waist region 56 and the second waist region 58.

While the elasticized side panels 30 may be constructed in a number of configurations, an example of a diaper with elasticized side panels positioned in the ears (ear flaps) of the diaper is disclosed in U.S. Patent 4,857,067, entitled "Disposable Diaper Having Shirred Ears" issued to Wood, et al. on August 15, 1989. The elasticized side panels 30 may alternatively be formed in a number of other configurations. For example, U.S. Patent 4,381,781 issued to Sciaraffa, et al. on May 3, 1983, discloses a diaper having an elasticized waist in which an elastic member is positioned in an opening in both the topsheet and the backsheet of the diaper such that the stretch of the elastic member will not be constrained by the non-elastic materials. While the Sciaffra et al. patent teaches the criticality of removing both the topsheet and the backsheet portions of the diaper in those areas coinciding with the elastic member, the present inventors have learned that satisfactory elastic performance can also be obtained when only one or when none of the coinciding portions of the topsheet and the backsheet are removed, especially when the portions of the diaper web containing the elastic member are subjected to an incremental mechanical stretching operation of the type described herein. A further embodiment of a diaper showing elasticized side panels is shown in U.S. Patent 4,938,753 issued to Van Gompel, et al. on July 3, 1990. This patent discloses a pant-like garment provided with stretchable side panels formed by attaching discrete stretchable members to the side edges of the main body of the garment. Thus, the elasticized side panels 30 of the present invention may comprise a separate elastically extensible material or laminate joined to the diaper. As shown in Figure 1, each elasticized side panel 30 preferably comprises an ear flap 88 and an elastic side panel member 90 operatively associated therewith that has been mechanically stretched to form a "zero strain" stretch laminate side panel. A more detailed description of a zero strain stretch laminate side panel and the methods and apparaus for making them are disclosed in the hereinbefore referenced U.S. Patent Application Serial No. 07/750,775 of Buell, et al. filed on August 22, 1991

The diaper 20 is preferably applied to a wearer by positioning one of the waist regions, preferably the second waist region 58, under the wearer's back and drawing the remainder of the diaper between the wearer's legs so that the other waist region, preferably the first waist region 56, is positioned across the front of the wearer. The tab portions 94 of the tape tabs 92 are then released from the release portion 95. The diaperer then wraps the elasticized side panel 30 around the wearer, while still grasping the tab portion 94. The elasticized side panel 30 will typically be extended and tensioned during this operation so as to conform to the size and shape of the wearer. The first fastening component 112, the adhesive attachment layer 96, is secured to the second fastening component 114 of the landing member 44 to effect a side closure. In the preferred embodiment of the present invention, when the side closure is formed, the waist closure is also "automatically" formed, i.e., the waist closure is passively activated. The waist closure is formed by the engagement of the first attachment components 46 with the second attachment component 48. With the formation of the waist closure, the elasticized waistband 35 is pretensioned so as to provide the fit and containment benefits described herein.

## Claims

1. A disposable absorbent article, preferably a diaper, (20) or incontinence garment, comprising
a liquid pervious topsheet (24);
a liquid impervious backsheet (26) joined with said topsheet (24);
an absorbent core (28) having side edges (82) and waist edges (83), said absorbent core (28) being positioned between said topsheet (24) and said backsheet (26); and
an elastic feature extending outwardly from one of said edges of said absorbent core (28), said elastic feature comprising a non-elastic web (77) and an elastomeric member (76) joined with said non-elastic web such that a portion of said non-elastic web (77) coincides with at least a portion of said elastomeric member (76)
characterised in that
said non-elastic web (77) comprises a segment of said topsheet (24) coinciding with said elastomeric member (76), said segment of said topsheet being mechanically prestrained such that said segment of said topsheet assumes a pattern of strained ribs (142) and unstrained gaps (144).

2. The absorbant article (20) of Claim 1 wherein said elastic feature comprises an elasticized leg cuff (32) extending laterally outwardly from one said side edges (82) of said absobent core (28).

3. The absorbent article (20) of Claim 1 wherein said elastic feature comprises an elastic waist feature (34) at least extending longitudinally outwardly from one of said waist edges (34) of said absorbent core (28).

4. The absorbent article (20) according to any of the preceding claims wherein said elastomeric member (76) comprises a heat shrinkable elastic material.

5. The absorbent article (20) according to any of the preceding claims wherein said elastic feature is a zero strain stretch laminate.

6. The absorbent article (20) according to any of the preceding claims additionally comprising elasticized side panels (30), each of said elasticized side panels (30), being elastically extensible in the lateral direction.

## Patentansprüche

1. Ein wegwerfbarer absorbierender Artikel, vorzugsweise eine Windel (20) oder ein Inkontinenz-Kleidungsstück, welcher umfaßt
ein flüssigkeitsdurchlässiges Deckblatt (24);
ein flüssigkeitsundurchlässiges Rückenblatt (26), welches mit dem genannten Deckblatt (24) verbunden ist;
einen absorbierenden Kern (28), welcher Seitenränder (82) und Taillenränder (83) aufweist, wobei der genannte absorbierende Kern (28) zwischen dem genannten Deckblatt (24) und dem genannten Rückenblatt (26) positioniert ist; und
ein elastisches Taillenelement, welches sich von einem der genannten Ränder des genannten absorbierenden Kerns (28) auswärts erstreckt, wobei das genannte elastische Element eine nicht-elastische Bahn (77) und einen elastomeren Bauteil (76), welcher mit der genannten nichtelastischen Bahn verbunden ist, umfaßt, sodaß ein Abschnitt der genannten nicht-elastischen Bahn (77) mit mindestens einem Abschnitt des genannten elastomeren Bauteils koinzidiert,
dadurch gekennzeichnet, daß die genannte nicht-elastische Bahn (77) ein Segment des genannten Deckblatts (24), welches mit dem genannten elastomeren Bauteil (76) konzidiert, umfaßt, wobei das genannte Segment des genannten Deckblatts mechanisch vorgedehnt ist, sodaß das genannte Segment des genannten Deckblatts ein Muster von gedehnten Rippen (142) und ungedehnten Zwischenräumen (144) annimmt.

2. Der absorbierende Artikel (20) nach Anspruch 1, bei welchem das genannte elastische Element ein elastifiziertes Beinbündchen (32) umfaßt, welches sich von einem der genannten Seitenränder (82) des genannten absorbierenden Kerns (28) seitlich auswärts erstreckt.

3. Der absorbierende Artikel (20) nach Anspruch 1, bei welchem das genannte elastische Element ein elastisches Taillenelement (34) umfaßt, welches sich von einem der genannten Taillenränder (34) des genannten absorbierenden Kerns (28) mindestens der Länge nach auswärts erstreckt.

4. Der absorbierende Artikel (20) nach einem der vorhergehenden Ansprüche, bei welchem der genannte elastomere Bauteil (76) ein Hitzeschrumpfbares elastisches Material umfaßt.

5. Der absorbierende Artikel (20) nach einem der vorhergehenden Ansprüche, bei welchem das genannte elastische Element ein spannungsloses Strecklaminat ist.

6. Der absorbierende Artikel (20) nach einem der vorhergehenden Ansprüche, welcher zusätzlich elastifizierte Seitenfelder (30) umfaßt, wobei jedes der genannten elastifizierten Seitenfelder in der Querrichtung elastisch verlängerbar ist.

## Revendications

1. Article absorbant à jeter après usage, de préférence une couche (20) ou un vêtement pour l'incontinence, comprenant :
une feuille de dessus perméable aux liquides (24) ;
une feuille de fond imperméable aux liquides (26) réunie avec ladite feuille de dessus (24) ;
une âme absorbante (28) ayant des bords de côté (82) et des bords de ceinture (83), ladite âme absorbante (28) étant disposée entre ladite feuille de dessus (24) et ladite feuille de fond (26) ; et
une structure élastique s'étendant a l'extérieur de l'un desdits bords de ladite âme absorbante (28), ladite structure élastique comprenant une bande non élastique (77) et un élément élastomère (76) réuni à ladite bande non élastique de telle sorte qu'une partie de ladite bande non élastique (77) coïncide avec au moins une partie dudit élément élastomère (76)
caractérisé en ce que,
ladite bande non élastique (77) comporte un segment de ladite feuille de dessus (24) coïncidant avec ledit élément élastomère (76), ledit segment de ladite feuille de dessus étant mécaniquement précontraint de telle sorte que ledit segment de ladite feuille de dessus prenne un motif de nervures contraintes (142) et d'intervalles non contraints (144).

2. Article absorbant (20) selon la revendication 1, dans lequel ladite structure élastique comporte un revers de jambe élastifié (32) s'étendant latéralement vers l'extérieur à partir de l'un desdits bords de côté (82) de ladite âme absorbante (28).

3. Article absorbant (20) selon la revendication 1, dans lequel ladite structure élastique comporte une structure de ceinture élastique (34) s'étendant au moins longitudinalement vers l'extérieur à partir de l'un des bords de ceinture (34) de ladite âme absorbante (28).

4. Article absorbant (20) selon l'une quelconque des revendications précédentes, dans lequel ledit élément élastomère (76) comporte un matériau élastique thermo-rétrécissable.

5. Article absorbant (20) selon l'une quelconque des revendications précédentes, dans lequel ladite structure élastique est un stratifié étirable à contrainte nulle.

6. Article absorbant (20) selon l'une quelconque des revendications précédentes, comprenant, en outre, des panneaux de côté élastifiés (30), chacun desdits panneaux de côté élastifiés (30) étant extensible élastiquement dans la direction latérale.
